# EUROPEAN PATENT APPLICATION

(11) **EP 2 204 159 A1**
(43) Date of publication of application: **07.07.2010**
(21) Application number: 08841609.4
(22) Date of filing: 15.10.2008
(51) Int. Cl.: A61K 8/29, A61K 8/19, A61K 8/39, A61K 8/81, A61K 8/891, A61Q 19/00

(54) **OIL-BASED SOLID COSMETIC PREPARATION**

(30) Priority: 23.10.2007 JP 2007274632
(71) Applicant: Shiseido Company, Ltd., Chuo-ku Tokyo 104-8010 (JP)
(72) Inventor: MASACHIKA, Kiriko, Yokohama-shi Kanagawa 224-8558 (JP)
(74) Representative: Green, Mark Charles
(86) International application number: PCT/JP2008/068681
(87) International publication number: WO 2009/054301

(57) **Abstract**

Disclosed is an oily solid cosmetic material, including
(a) 30 - 50% by mass of a titanium oxide(s),
(b) 5 - 30% by mass of an iron-oxide-type pigment(s), and
(c) 2 - 5% by mass of a diglyceryl diisostearate(s).

## Description

### TECHNICAL FIELD

The present invention relates to an oily solid cosmetic material.

### BACKGROUND ART

In order to hide a damaged skin portion such as a skin scar, a scar or the like has conventionally been covered and made less noticeable by thickly applying a cosmetic material thereon.

However, a cosmetic material that has generally been used is frequently not able to heal a damaged skin such as a skin scare, depending on the extent thereof, and a cosmetic material that is able to hide a damaged skin portion more effectively has been desired.

Meanwhile, a titanium oxide powder, which may be used in an embodiment of the present invention, has been used as a powder for cosmetic material, wherein it has been provided, for example, in practical example 3 of Japanese Patent Application Publication No. 08-081331 (patent document 1) and in practical examples 1 and 2 of Japanese Patent Application Publication No. 07-089826 (patent document 2) for formulations of multiple compounds but there has been no consideration that a sufficient effect of repairing a damaged skin portion is provided thereby.

Furthermore, it is essential to compound an iron-oxide-type pigment into a foundation or the like in order to provide a color thereto, but, when a high amount of titanium oxide is compounded into such a system, the melting viscosity becomes high, whereby the dispersion property of a coloring material is degraded and a disadvantage of generating a colored fringe occurs.

### DISCLOSURE OF THE INVENTION

### MEANS FOR SOLVING THE PROBLEM

According to one aspect of the present invention, there is provided an oily solid cosmetic material, including
(a) 30 - 50% by mass of a titanium oxide(s),
(b) 5 - 30% by mass of an iron-oxide-type pigment(s), and
(c) 2 - 5% by mass of a diglyceryl diisostearate(s).

### BEST MODE FOR CARRYING OUT THE INVENTION

The best mode of an embodiment of the present invention will be described below.

It is possible for an embodiment of the present invention to provide an oily solid cosmetic material that has an ability to cover a damaged skin portion, is excellent in the effect of repairing a damaged skin portion, is excellent in the dispersion property of a coloring material, provides a sufficient coloring level, and prevents colored fringe.

The inventors have widely investigated for an oily solid cosmetic material that is excellent in the effect of repairing a damaged skin portion and prevents colored fringe, and as a result, have found that a particular base powder and a particular dispersing agent are used.

An embodiment of the present invention is, for example, an oily solid cosmetic material characterized by containing (a) 30 - 50% by mass of a titanium oxide(s), (b) 5 - 30% by mass of an iron-oxide-type pigment(s), and (c) 2 - 5% by mass of a diglyceryl diisostearate(s). An oily solid cosmetic material according to an embodiment of the present invention may preferably be used as a cosmetic material for repairing a damaged skin.

An oily solid cosmetic material according to an embodiment of the present invention may have a covering ability, be excellent in the effect of repairing a damaged skin portion, provide a good dispersion property of a coloring material, and prevent colored fringe.

A titanium oxide(s) as a component(s) to be used in an embodiment of the present invention is/are, for example, a pigment-grade titanium oxide(s) whose average particle size is 0.3 µm or more. The compounding amount is, for example, 30 - 50% by mass, and more preferably, 35 - 45% by mass. If it is less than 30% by mass, the covering ability may be degraded, and accordingly, the effect of repairing a damaged skin may be insufficient, while, if it is more than 50% by mass, a coloring material may not provide a sufficient coloring level and the thickness feeling may be enhanced excessively resulting in an unnatural feeling.

For an iron-oxide-type pigment(s) as a component (b) to be used in an embodiment of the present invention, it is possible to provide, for example, an iron oxide (red iron oxide), iron titanate, and the like, which are red color pigments; γ-iron oxide, which is a brown color pigment; yellow iron oxide, which is a yellow color pigment; and black iron oxide, which is a black color pigment. One kind or two or more kinds of these may be selected and used appropriately.

For the amount of an iron-oxide-type pigment(s) compounded in an oily solid cosmetic material according to an embodiment of the present invention, it is possible to compound, for example, 5 - 30% by mass, and preferably, 6 - 26% by mass thereof into the composition. In such a range, it is possible to provide an appropriate coloring level.

In an embodiment of the present invention, it is preferable that the compounding ratio (mass ratio) of (a) a titanium oxide(s) to (b) an iron-oxide-type pigment(s) is, for example, (a):(b) = 6:1 - 1:1. In such a range, both a covering ability and a coloring level may be good.

(c) a diglyceryl diisostearate(s) to be used in an embodiment of the present invention is represented by, for example, the following formula (1), which may be a single one or a mixture thereof. When a dispersing agent other than a diglyceryl diisostearate(s) is used, a good dispersion property of an iron-oxide-type pigment(s) may not be obtained in a system in which a high amount of a titanium oxide(s) is/are compounded.

RO-[CH₂CH(OR)CH₂O]ₙ-R (1),

(in which formula, R represents H or an isostearate residue and the average number of isostearate residue in one molecule thereof is 2, and the average number of n is 2)

For the amount of diglyceryl diisostearate compounded in an oily solid cosmetic material according to an embodiment of the present application, for example, 2 - 5% by mass thereof is compounded in the composition. If the amount of compounded diglyceryl diisostearate is less than 2% by mass, a colored fringe may be generated even if a small amount of an iron-oxide-type pigment(s) is provided, while if it is more than 5% by mass, an appropriate feeling of fit at the point of use may be lost or a covering ability may be degraded.

In an embodiment of the present invention, a coating material including (d) an acryl-silicone-type graft copolymer and a silicone-treated polysaccharide compound is used whereby it is possible to conduct additional application readily so that the effect of repairing a damaged skin portion may be enhanced and makeup may be held to last longer. These coating materials will be described in detail below.

An acryl-silicone-type graft copolymer is a copolymer of an organopolysiloxane compound having a radical-polymerizable group at one end of the molecular chain thereof and a radical polymerizable monomer based on an acrylate and/or a methacrylate, and examples thereof are as disclosed in Japanese Patent Application Publication No. 02-025411 and Japanese Patent Application Publication No. 02-132141. For an organopolysiloxane compound having a radical-polymerizable group at one end of the molecular chain thereof, it is possible to provide, for example, ones represented by the following general formula (2): 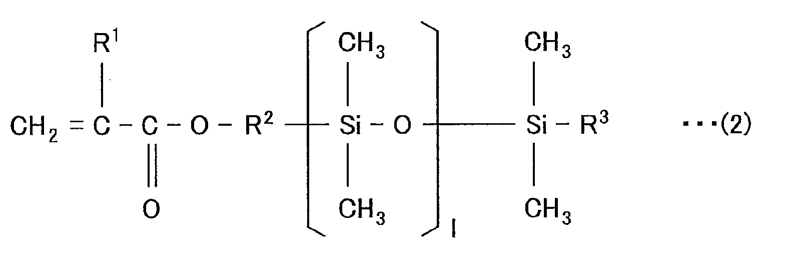 wherein there are provided R¹: a methyl group or a hydrogen atom, R²: a divalent saturated hydrocarbon group with 1 - 10 carbon atoms which has a linear or branched carbon chain that may be interposed by one or two ether linkages, R³: a methyl group or a butyl group, and 1: 3 - 300.

On the other hand, a radical-polymerizable monomer based on an acrylate and/or a methacrylate means a compound having one radical-polymerizable unsaturated bond in the molecule thereof, and for examples of an acrylate and/or a methacrylate to be used, it is possible to provide alkyl (meth)acrylates such as methyl (meth)acrylate, ethyl (meth)acrylate, n-butyl (meth)acrylate, 2-ethylhexyl (meth)acrylate, stearyl (meth)acrylate, and behenyl (meth)acrylate; hydroxylalkyl (meth)acrylates such as 2-hydroxylethyl (meth)acrylate and 2-hydroxypropyl (meth)acrylate; and perfluoroalkyl (meth)acrylates with 1 - 10 fluorocarbon chains. Furthermore, for a radical-polymerizable monomer based on an acrylate and/or a methacrylate in an embodiment of the present invention, it is possible to use various kinds of polymerizable monomer compounds according to need, besides the acrylates and/or methacrylates as described above. For examples of these compounds, it is possible to provide styrene, substituted styrenes, vinyl acetate, (meth)acrylic acid, maleic anhydride, maleic acid esters, fumaric acid esters, vinyl chloride, vinylidene chloride, ethylene, propylene, butadiene, acrylonitrile, fluorinated olefins, N-vinyl pyrolidone, and the like.

Manufacturing of a copolymer of (A) a dimethylpolysiloxane compound having a radical-polymerizable group at one end of the molecular chain thereof and (B) a radical-polymerizable monomer based on an acrylate and/or methacrylate is conducted, for example, within a range of a polymerization ratio ((A)/(B)): 1/19 - 2/1 under the presence of a conventional radical-polymerization initiator such as benzoyl peroxide, lauroyl peroxide, azobisisobutyronitrile, wherein it is also possible to apply any method of a solution polymerization method, an emulsion polymerization method, a suspension polymerization method, and a bulk polymerization method. For commercial products thereof, it is possible to provide, for example, KP541, KP543, KP545 (all produced by Shin-Etsu Chemical Co., Ltd.), and the like, as being dissolved in a solvent.

It is possible to use one kind or two or more kinds of these acryl-silicone-type graft copolymers according to need.

A silicone-treated polysaccharide compound to be used in an embodiment of the present invention is represented by, for example, the following general formula (3). 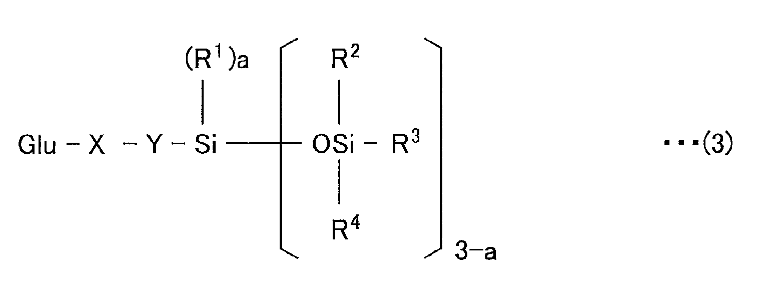 (in which formula, Glu, X, and Y represent a saccharide residue of a polysaccharide compound, a divalent linkage group, and a divalent aliphatic group, respectively, and R¹ and each of R², R³, and R⁴ represent a monovalent organic group with a carbon number of 1 - 8 and a monovalent organic group with a carbon number of 1 - 8 or a siloxy group represented by -OsiR⁵R⁶R⁷. Herein, each of R⁵, R⁶, and R⁷ represents a monovalent organic group with a carbon number of 1 - 8 and "a" represents 0, 1, or 2.)

In the general formula (3), Glu represents a saccharide residue of a polysaccharide compound, wherein, for such a polysaccharide compound, it is possible to use publicly-known various kinds of polysaccharide compounds, and it is possible to provide, for example, celluloses, hemicelluloses, gum arabic, tragacanth gum, tamarind gum, pectin, starches, mannans, guar gum, locust bean gum, Quince seed gum, alginic acid, carageenan, agar, xanthan gum, dextran, pullulan, chitin, chitosan, hyaluronic acid, chondroitin sulfate, as well as derivatives of these polysaccharide compounds, for example, polysaccharide compound derivatives having been subjected to carboxymethylation, sulfation, phosphorylation, methylation, ethylation, addition of an alkylene oxide such as ethylene oxide or propylene oxide, acylation, cationization, molecular weight reduction, or the like. Among these, ethylcellulose or pullulan is preferable and pullulan is particularly preferable. Additionally, the average molecular weight of a polysaccharide compound in an embodiment of the present invention is dependent on the kind of a polysaccharide compound, and commonly, about 1,000 - 5,000,000 is preferable.

Such a polysaccharide compound contains at least one or more of one kind or two or more kinds of reactive functional groups such as a hydroxyl group and a carboxyl group depending on the kind thereof. A divalent linkage group represented by "X" is a linkage group which is derived from "A" and formed by reacting a reactive functional group possessed by such a polysaccharide compound with a silicone compound represented by the following general formula (4). Additionally, it is possible to use a conventionally- and publicly-known method for such reaction of a polysaccharide compound with a silicone compound.

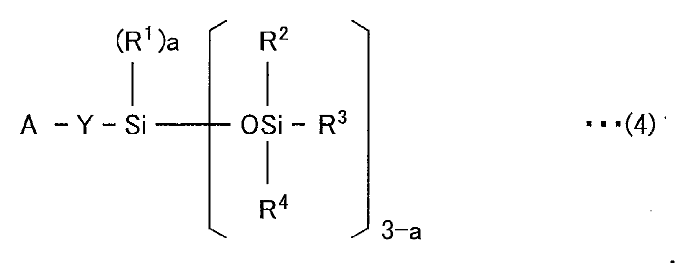

In the formula described above, Y, R¹, R², R³, R⁴, and "a" are identical to those of the general formula (3) described above. Furthermore, "A" is a functional group capable of reacting with a reactive functional group of a polysaccharide compound, and it is possible to provide, for example, an isocyanate group, an epoxy group, a vinyl group, an acryloyl group, a methacryloyl group, an amino group, an imino group, a hydroxyl group, a carboxyl group, a mercapto group, and the like.

As examples of "X", it is possible to provide a carbamoyl group, -CH₂CH(OH)-, a carbonyl group, an amino group, an ether group, and the like, and from the viewpoint of reactivity, a carbamoyl group (-CONH-) is preferable which is formed by reacting a compound of the general formula (4) wherein "A" is an isocyanate group (O=C=N-), with an hydroxyl group of a polysaccharide compound. Additionally, in this case, a saccharide residue of a polysaccharide compound means a residual part of a polysaccharide compound in which a hydrogen group of a hydroxyl group that reacts with an isocyanate group is excluded. Furthermore, in cases of other reactions, a saccharide residue of a polysaccharide compound also means an equivalent to this one.

For divalent aliphatic groups represented by "Y", it is possible to provide, for example, alkylene groups, alkylene groups having an oxygen atom, a nitrogen atom, a sulfur atom, or the like in the main chain thereof, alkylene groups having an arylene group such as a phenylene group in the main chain thereof, and alkylene groups having a carbonyloxy group or an oxycarbonyl group in the main chain thereof. It is possible for these divalent aliphatic groups to have, for example, a substituent such as a hydroxy group, an alkoxy group, or an alkyl group, and a terminal atom of the aliphatic group may be a hetero atom such as an oxygen atom, a nitrogen atom, or a sulfur atom. As examples of "Y", it is possible to provide -(CH₂)₂-, - (CH₂)₃-, -(CH₂)₄-, -(CH₂)₆-, -(CH₂)₈-, -[CH₂CH(CH₃)]-, - (CH₂)₂O(CH₂)₃-, -CH₂CH(OH)-CH₂-, and the like, and a propylene group represented by -(CH₂)₃- is preferable.

For examples of a monovalent organic group with a carbon number of 1 - 8 provided for R¹, R², R³, R⁴, R⁵, R⁶, and R⁷ in the general formula (3) described above, it is possible to provide alkyl groups such as a methyl group, an ethyl group, a propyl group, and a butyl group; cycloalkyl groups such as a cyclopentyl group and a cyclohexyl group; aryl groups such as a phenyl group; aralkyl groups such as a benzyl group; alkenyl groups such as a vinyl group and an allyl group; fluorinated alkyl groups such as 3,3,3-trifluoropropyl group; and the like.

Furthermore, each of R², R³, and R⁴ may be a siloxy group represented by -OSiR⁵R⁶R⁷. For examples of such a siloxy group, there are provided a trimethylsiloxy group, an ethyldimethylsiloxy group, a phenyldimethylsiloxy group, a vinyldimethylsiloxy group, 3,3,3-trifluoropropyldimethylsiloxy group, and the like. Additionally, R¹, R², R³, R⁴, R⁵, R⁶, and R⁷ may be identical or different, and "a" = 0 and R², R³, and R⁴ being methyl groups are particularly preferable for a silicone-treated polysaccharide compound in an embodiment of the present invention.

For silicone-treated polysaccharide compounds to be used in an embodiment of the present invention, silicone-treated pullulans represented by the following formula (5) are particularly preferable. Additionally, "PL" in the formula (5) represents a glucose residue of a pullulan.

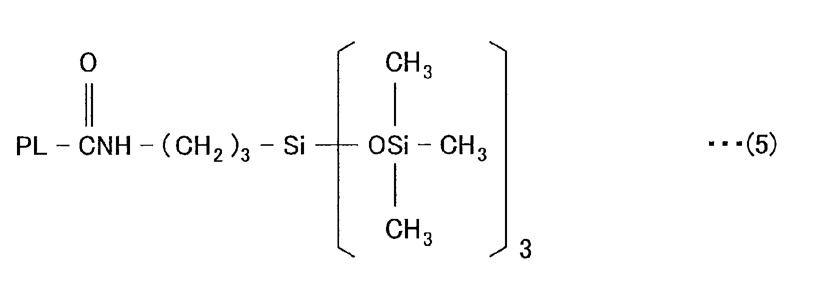

Additionally, in a silicone-treated polysaccharide compound according to an embodiment of the present invention, the rate of a silicone compound bonding to a reactive functional group of a polysaccharide compound is dependent on the kind thereof, and it is usually preferable that the average number (substitution degree) of a silicone compound bonding to 1 unit of constitutional saccharide of a polysaccharide compound be 0.5 - 3.0. Additionally, such a substitution degree in an embodiment of the present invention is equivalently calculated from an Si content (% by mass) in such a compound.

Additionally, when a silicone-treated polysaccharide compound is compounded by dissolving it in a low-molecular-weight silicone oil or a light iso-paraffin, it may be possible to enhance the facilitation of compounding, the usability, or the like.

It is possible to use one kind or two or more kinds of these silicone-treated polysaccharide compounds according to need. Furthermore, it is possible to provide, for example, TSPL=30-D5 (produced by Shin-Etsu Chemical Co., Ltd., 30% by weight solution in decamethylcyclopentasiloxane) for a commercial product of a silicone-treated polysaccharide compound.

For the amount of a coating material including an acryl-silicone-type graft copolymer and a silicone-treated polysaccharide compound which material is compounded in an oily solid cosmetic material according to an embodiment of the present invention, it is possible to compound, for example, 2 - 20% by weight, and preferably 2 - 15% by weight thereof into a composition. If the amount of a compounded coating material is excessively high, the spreadability may be degraded or the tensile stress may be generated after a finish.

Furthermore, a coating material is preferably composed of an acryl-silicone-type graft copolymer(s) and a silicone-treated polysaccharide compound(s), and it is possible to contain a coating material(s) other than the aforementioned one, if it is 10% by mass or less of the entire coating material(s).

For such coating materials, it is possible to provide, for example, polyvinylpyrolidone/a-olefin copolymers, trimethylsiloxysilicate polymers, and amino-modified silicones. However, if the amount of such a coating material(s) is excessively high, a disadvantage may be caused such that the facilitation of additional application or the makeup lasting may be deteriorated or the tensile stress may be sensed.

For the compounding rate (mass ratio) of an acryl-silicone-type graft copolymer(s) to a silicone-treated polysaccharide compound(s), an acryl-silicone-type graft copolymer(s): a silicone-treated polysaccharide compound(s) = 1:0.1 - 1:0.3 is preferable.

If the compounding rate of an acryl-silicone-type graft copolymer(s) is excessively high, the flexibility may be degraded, additional application may become difficult, or the makeup lasting may be deteriorated. Furthermore, if the rate of a silicone-treated polysaccharide compound(s) is excessively high, the stickiness may be generated, the usability may be degraded, or fitting on skin may become poor.

In an embodiment of the present invention, it is preferable to further contain a calcined mica other than the components described above. When a calcined mica is compounded, the spreadability may be improved so as to improve the usability. A desired amount of a calcined mica to be compounded is, for example, 40% by mass or less, and preferably 5 - 20% by mass, of the total amount of an oily solid cosmetic material.

For other powders in an embodiment of the present invention, it is possible to compound, for example, an inorganic powder of talc, kaolin, mica, sericite, muscovite, biotite, phlogopite, a synthetic mica, vermiculite, magnesium carbonate, calcium carbonate, an aluminum silicate, a barium silicate, a calcium silicate, a magnesium silicate, a metal tungstate, magnesium, silica, a zeolite, barium sulfate, a calcined calcium sulfate, a calcined gypsum, calcium phosphate, fluoroapatite, hydroxylapatite, a ceramic powder, or a metal soap (such as zinc myristate, calcium palmitate, or aluminum stearate); an organic powder such as a polyamide resin powder, a polyethylene powder, a poly(methyl methacrylate) powder, a polystyrene powder, a styrene-acrylic acid copolymer resin powder, a benzoguanamine resin powder, a polytetrafluoroethylene powder, or a cellulose powder; an inorganic whitish pigment such as zinc oxide; an inorganic yellowish pigment such as loess; an inorganic blackish pigment such as carbon or low titanium oxide; an inorganic purplish pigment such as mango violet or cobalt violet; an inorganic greenish pigment such as chromium oxide, chromium hydroxide, or cobalt titanate; an inorganic bluish pigment such as ultramarine blue or iron blue; a pearl pigment such as a titanium-oxide-coated mica, a titanium-oxide-coated bismuth oxychloride, a titanium-oxide-coated talc, a colored-titanium-oxide-coated mica, bismuth oxychloride, or pearl essence; a metal powder pigment such as an aluminum powder or a copper powder; an organic pigment such as Red No. 202, Red No. 205, Red No. 220, Red No. 228, Red No. 405, Orange No. 203, Orange No. 204, Yellow No. 205, Yellow No. 401, or Blue No. 404; an organic pigment such as a zirconium, barium or aluminum lake such as Red No. 3, Red No. 104, Red No. 227, Red No. 401, Orange No. 205, Yellow No. 4, Yellow No. 202, Green No. 3, or Blue No. 1; a natural color such as a chlorophyll or β-carotene; or the like.

Furthermore, it is possible to compound a component that is commonly used for a cosmetic material into an oily solid cosmetic material according to an embodiment of the present invention, unless the effect of an embodiment of the present invention is impaired. For example, it is possible to compound into a composition according to an embodiment of the present invention, a humectant such as a polyhydric alcohol, a mucopolysaccharide (such as sodium hyaluronate) or an organic acid or organic salt (such as an amino acid, a salt of amino acid, or a salt of oxyacid), for an aqueous phase component, or a solid or semisolid oil component such as vaseline, lanolin, a silicone wax, a higher fatty acid or a higher alcohol, a fluid oil component such as scwaran, a liquid paraffin, an ester oil, a triglyceride, a volatile hydrocarbon oil or a fluorocarbon, a surfactant such as a cationic surfactant, an anionic surfactant or a non-ionic surfactant, a drug such as vitamin E or vitamin E acetate, an astringent, an antioxidant, an antiseptic, a flavor, a pH adjuster such as dibasic sodium phosphate, a clay mineral, a thickener, or an ultraviolet absorber, for an oil phase component, or the like.

An oily solid cosmetic material according to an embodiment of the present invention is preferably used as, for example, a foundation, a makeup base, or a concealer (partial base).

Next, practical examples of an embodiment of the present invention will be described. The present invention shall not be limited to them. Compounding amounts are all provided in % by mass.

Practical examples 1 - 7 and comparative examples 1 - 4

### [Formulations]

Oily solid cosmetic materials were manufactured in accordance with formulations illustrated in the following Table 1 and Table 2 and their effects were evaluated by an evaluation method described below. The results are illustrated in Table 1 and Table 2 integrally.

**Table 1**

| Test examples | | Practical examples | | | | | | |
|---|---|---|---|---|---|---|---|---|
| | | 1 | 2 | 3 | 4 | 5 | 6 | 7 |
| Titanium oxide | Titanium oxide | 30 | 30 | 50 | 30 | 30 | 50 | 30 |
| Lamellar powder | Baked mica | 15 | 15 | 5 | 15 | 15 | 5 | 15 |
| Iron-oxide-type pigments | Red iron oxide | 1 | 1.5 | 5.5 | 1.5 | 5.5 | 1.5 | 1.5 |
| | Yellow iron oxide | 3.8 | 5.2 | 12 | 5.2 | 12 | 5.2 | 5.2 |
| | Black iron oxide | 0.2 | 0.3 | 2.5 | 0.3 | 2.5 | 0.3 | 0.3 |
| Dispersing agents | Diglyceryl diisostearate | 2 | 2 | 2 | 5 | 2 | 2 | 2 |
| | Sorbitan sesqui-isostearate | 3 | 3 | 3 | - | 3 | 3 | - |
| Coating agents | Acryl-silicone-type graft copolymer | 1 | 1 | 1 | 1 | 1 | 1 | 1 |
| | Silicone-treated polysaccharide compound (30 wt% decamethyl-cyclopentasiloxane solution) | 1 | 1 | 1 | 1 | 1 | 1 | 1 |
| Volatile oil component | Decamethyl-cyclopentasiloxane | 15 | 15 | 10 | 15 | 5 | 5 | 18 |
| Waxes | Ceresin | 2 | 2 | 2 | 2 | 2 | 2 | 2 |
| | Polyethylene wax (average molecular weight of 500) | 4 | 4 | 4 | 4 | 4 | 4 | 4 |
| Oil components | Squalane | 12 | 10 | 1 | 10 | 7 | 10 | 10 |
| | Dimethyl-polysiloxane | 10 | 10 | 1 | 10 | 10 | 10 | 10 |
| | Total | 100 | 100 | 100 | 100 | 100 | 100 | 100 |
| Dispersion property of coloring material (degree of colored fringe absence) | | A | A | B | A | B | B | C |
| Covering ability | | B | B | A | B | B | A | B |
| Coloring level | | B | A | B | A | A | D | A |

**Table 2**

| Test examples | | Comparative examples | | | |
|---|---|---|---|---|---|
| | | 1 | 2 | 3 | 4 |
| Titanium oxide | Titanium oxide | 30 | 30 | 15 | 30 |
| Lamellar powder | Baked mica | 15 | 15 | 15 | 15 |
| Iron-oxide-type pigments | Red iron oxide | 1 | 1 | 1 | 0.45 |
| | Yellow iron oxide | 3.8 | 3.8 | 3.8 | 2.5 |
| | Black iron oxide | 0.2 | 0.2 | 0.2 | 0.05 |
| Dispersing agents | Diglyceryl diisostearate | - | - | 3 | 3 |
| | Sorbitan sesqui-isostearate | 3 | 5 | - | - |
| Coating agents | Acryl-silicone-type graft copolymer | 1 | 1 | 1 | 1 |
| | Silicone-treated polysaccharide compound (30 wt% decamethyl-cyclopentasiloxane solution) | 1 | 1 | 1 | 1 |
| Volatile oil component | Decamethyl-cyclopentasiloxane | 15 | 13 | 15 | 15 |
| Waxes | Ceresin | 2 | 2 | 2 | 2 |
| | Polyethylene wax (average molecular weight of 500) | 4 | 4 | 4 | 4 |
| Oil components | Squalane | 14 | 14 | 29 | 16 |
| | Dimethylpolysiloxane | 10 | 10 | 10 | 10 |
| | Total | 100 | 100 | 100 | 100 |
| Dispersion property of coloring material (degree of colored fringe absence) | | E | D | B | D |
| Covering ability | | B | B | E | B |
| Coloring level | | B | B | A | E |

(Method for manufacturing oily solid cosmetic materials in Table 1 and Table 2)

Respective ingredients are heated and melted at 90 - 100 °C, agitated and mixed, degassed, and cooled down.

### [Evaluation method]

Expert panelists (6 persons) used the oily solid cosmetic materials manufactured in practical examples 1 - 7 and comparative examples 1 - 4 described above, conducted 5-grade sensory evaluations with respect to the dispersion property of a coloring material (the degree of absence of a colored fringe), a covering ability, and a coloring level (the degree of coloring), and made determinations by the following evaluation criteria.

### [Evaluation criteria]

5: Very good
4: Good
3: Normal
2: Bad
1: very bad

### [Evaluations]

A: Evaluation value (mean value) 4.5 or more
B: Evaluation value (mean value) 3.5 or more and less than 4.5
C: Evaluation value (mean value) 2.5 or more and less than 3.5
D: Evaluation value (mean value) 1.5 or more and less than 2.5
E: Evaluation value (mean value) less than 1.5

As can be seen from Table 1 and Table 2, an oily solid cosmetic material according to an embodiment of the present invention is excellent in the dispersion property of a coloring material (the degree of absence of a colored fringe), a covering ability, and a coloring level.

### [Appendix]

Appendix (1): An oily solid cosmetic material characterized by containing (a) 30 - 50% by mass of a titanium oxide(s), (b) 5 - 30% by mass of an iron-oxide-type pigment(s), and (c) 2 - 5% by mass of a diglyceryl diisostearate(s).

Appendix (2): The oily solid cosmetic material as described in appendix (1), characterized by further containing a coating material including (d) an acryl-silicone-type graft copolymer and a silicone-treated polysaccharide compound.

Appendix (3): The oily solid cosmetic material as described in appendix (1) characterized by being a cosmetic material for repairing a damaged skin.

Although the embodiment(s) and practical example(s) of the present invention have specifically been described above, the present invention is not limited to the embodiment(s) or practical example(s) described above and it is possible to alter or modify the embodiment(s) or practical example(s) of the present invention without departing from the spirit and scope of the present invention.

### INDUSTRIAL APPLICABILITY

It is possible to apply an embodiment of the present invention to an oily solid cosmetic material. In particular, it is possible to apply an embodiment of the present invention to an oily solid cosmetic material with a function of healing a damaged skin portion such as a skin scar.

The present application claims the benefit of the priority based on Japanese Patent Application No. 2007-274632 filed on October 23, 2007, the entire content of which is hereby incorporated by reference.

## Claims

1. An oily solid cosmetic material, comprising
(a) 30 - 50% by mass of a titanium oxide(s),
(b) 5 - 30% by mass of an iron-oxide-type pigment(s), and
(c) 2 - 5% by mass of a diglyceryl diisostearate(s).

2. The oily solid cosmetic material as claimed in claim 1, further comprising
(d) an acryl-silicone-type graft copolymer and a silicone-treated polysaccharide compound.
